# EUROPEAN PATENT SPECIFICATION

(11) **EP 0 859 630 B1**
(45) Date of publication and mention of the grant of the patent: **11.12.2002**
(21) Application number: 96935971.0
(22) Date of filing: 01.10.1996
(51) Int. Cl.: A61K 38/21

(54) **COMBINATION OF TEMOZOLOMIDE AND ALPHA-IFN FOR THE TREATMENT OF ADVANCED CANCER**
KOMBINATION VON TEMOZOLOMIDE UND ALPHA-INTERFERON ZUR BEHANDLUNG VON FORTGESCHRITTENEM KREBS
COMBINAISON DE TEMOZOLOMIDE ET D'INTERFERON ALPHA POUR LE TRAITEMENT DU CANCER AVANCE

(30) Priority: 04.10.1995 US 6233 P; 27.03.1996 US 625410
(43) Date of publication of application: 26.08.1998
(73) Proprietor: SCHERING CORPORATION, Kenilworth New Jersey 07033 (US)
(72) Inventor: DUGAN, Margaret, H., Woodside, NY 11377 (US)
(74) Representative: Ritter, Stephen David
(86) International application number: US9615413
(87) International publication number: WO97012630

(56) References cited:
- WO-A-94/15651
- JOURNAL OF CLINICAL ONCOLOGY, 13 (4). 30-03-1995. 910-913., XP000612568 BLEEHEN N M ET AL: "Cancer research campaign phase II trial of temozolomide in metastatic melanoma"
- CANCER (PHILADELPHIA), 74 (3 SUPPL.). 1994. 1164-1176., XP000617951 ABRAMS J S ET AL: "New chemotherapeutic agents for breast cancer"
- PROC ANNU MEET AM ASSOC CANCER RES;35:A1769 1994, XP000617946 CARTER CA ET AL: "Responses of human melanoma, ovarian, and colon tumor xenografts in nude mice to oral temozolomide (Meeting abstract)."

## Description

Despite the numerous advances in cancer treatment, the well-known life style changes that can greatly reduce the risk of cancer, and the early warning signs that some cancers provide, many patients still develop advanced cancer for which no conventional therapies are available that offer any reasonable hope of cure or significant palliation. This invention is the use of two known anti-tumor agents in combination therapy to provide a positive effect on such advanced cancers. It is also expected that the combination therapy will allow the administration of the two anti-tumor agents in quantities that will not result in intolerable side effects.

Temozolomide is known for its anti-tumor effects. For example, in one study clinical responses were achieved in 17% of patients having advanced melanoma (Newlands ES, et al. Br J Cancer 65 (2) 287-2981, 1992). In another sudy a clinical response was achieved in 21% of patients with advanced melanoma (Journal of Clinical Oncology, Vol 13, No. 4 (April), 1995, pp 910-913). However, temozolomide is not always effective and has dose-limiting side effects, such as hematologic toxicity, myelosuppression, anemia, leukopenia, etc.

Alpha interferon is also known to have anti-cancer effects. See, for example, Ernstoff et al., Intravenous (IV) Recombinant α-2 Interferon in Metastatic Melanoma, Proc ASCO 2:57 (C-222), 1983. However this treatment is not always effective and sometimes results in intolerable side effects related to the dosage and duration of therapy.

There is a need for a method for treating advanced cancers with higher response rates or reduced side effects, or both.

### SUMMARY OF THE INVENTION

This invention may be summarized as the use of temozolomide (alpha interferon) in the manufacture of a medicament for use in the treatment of cancer in a combination therapy in which alpha interferon (temozolomide) is also to be administered.

Temozolomide and alpha interferon are to be administered in amounts sufficient to achieve a clinical response. The temozolomide is administered to the patient in combination with the alpha interferon, that is, the temozolomide and alpha interferon doses are administered during the same period of time. Preferred specific dosing schedules are given below.

### DETAILED DESCRIPTION

The term "temozolomide" is intended to mean a compound having the formula.

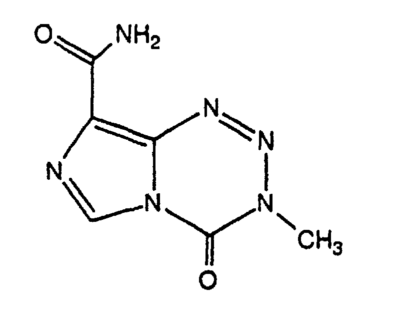

One chemical name for temozolomide is 3,4-dihydro-3-methyl-4-oxoimidazo-[5,1-d]1,2,3,4-tetrazin-8-carboximide. The synthesis of temozolomide is well known. See, for example, Stevens et al., J. Med. Chem, 1984, 27, 196-201 and Wang et al., J. Chem. Soc., Chem. Commun., 1994, pp 1687-1688.

The term "alpha interferon" as used herein means the family of highly homologous species-specific proteins that inhibit viral replication and cellular proliferation and modulate immune response. Typical suitable alpha interferons include but are not limited to recombinant interferon alpha-2b such as Intron-A interferon available from Schering Corporation, Kenilworth, N.J., recombinant interferon alpha-2a such as Roferon A interferon available from Hoffmann-La Roche, Nutley, N.J., recombinant interferon alpha-2C such as Berofor alpha 2 interferon available from Boehringer Ingelheim Pharmaceutical, Inc., Ridgefield, CT., interferon alpha-n1, a purified blend of natural alpha interferons such as Sumiferon available from Sumitomo, Japan or as Wellferon interferon alpha-n1 (INS) available from the Glaxo-Wellcome Ltd., London, Great Britain, or a consensus alpha interferon available from Amgen, Inc., Newbury Park, CA, or interferon alpha-n3 a mixture of natural alpha interferons made by Interferon Sciences and available from the Purdue Frederick Co., Norwalk, CT., under the Alferon Tradename. The use of interferon alpha-2a or alpha 2b is preferred. Interferon alpha 2b is most preferred. The manufacture of interferon alpha 2b is described in U.S. Patent No. 4,530,901. Of course the term alpha interferon includes the obvious equivalents thereto such as certain beta interferons known to have properties similar to alpha interferon.

Advanced cancers treatable by this invention include malignant melanoma, malignant metastasized melanoma, cancer of the lung, cancer of the breast, brain cancer, ovarian cancer, cancer of the head and/or neck, sarcoma, prostate cancer, and other cancers known to be at least partially responsive to alpha interferon or temozolomide treatment, that have advanced to a stage where conventional therapy is unlikely to provide a cure.

A person suffering from advanced cancer may exhibit one or more of the following signs or symptoms:
(a) presence of cancerous tumor,
(b) fatigue,
(c) pain,
(d) decreased performance status from tumor burden, and
(e) the well known symptoms associated with each specific cancer.
To practice the invention, temozolomide and alpha interferon are administered to the patient exhibiting one of more of the above signs or symptoms in amounts sufficient to eliminate or at least alleviate one or more of the signs or symptoms.

The preferred dosage of temozolomide for practicing the combination therapy of this invention is 50 to 400 mg per m² of the patient's body surface area per day, more preferably 75 to 300 mg/m² and most preferably 100 to 200 mg/m²/day. It is preferred that the daily dosage of temozolomide be administered once per day for a 2 to 10 day period, more preferably for a 3 to 8 day period and most preferably for a 5 day period. The temozolomide dosing periods may be repeated in cycles of 28 to 42 days, more perferably 28 to 35 days, and most preferably 28 days. That is, 28 to 42 days after the first day of temozolomide administration, another temozolomide administration period may be started.

Alternatively the temozolomide may be administered for a much longer period at reduced dosage. For example, the temozolomide could be administered daily for 11 days to six weeks at a dosage of 50 to 150 mg/m²/day.

Temozolomide may be administered orally in capsule form wherein it is admixed with conventional pharmaceutical carriers. Preferred temozolomide capsule formulations are:

| Ingredient | mg/Capsule | | | |
|---|---|---|---|---|
| temozolomide | 5 | 20 | 100 | 250 |
| Anhydrous Lactose NF | 132.8 | 182.2 | 175.7 | 154.3 |
| Sodium Starch Glycolate NF | 7.5 | 11.0 | 15.0 | 22.5 |
| Colloidal Silicon Diozide NF | 0.2 | 0.2 | 0.3 | 0.7 |
| Tartaric Acid NF | 1.5 | 2.2 | 3.0 | 9.0 |
| Steric Acid NF | 3.0 | 4.4 | 6.0 | 13.5 |
| Capsule Size* | 3 | 2 | 1 | 0 |

| | | | | |
|---|---|---|---|---|
| * White opaque, preservative-free, two-piece hard gelatin capsules | | | | |

It is especially preferred that the patient fast from all food or drink, except water, for four hours before temozolomide administration and for two hours after.

The alpha interferon is preferably administered by intravenous or subcutantous injection beginning on day one of the first temozolomide administration period. However, unlike the temozolomide, the alpha interferon is administered more or less regularly throughout the combination therapy. The alpha interferon may be administered 1 to 7 times per week, more preferably 2 to 5 times per week, and most preferably three times per week or every other day. The amount of alpha interferon per dose may be 1 million to 25 million international units (IU) per m² of patient's body surface area, more preferably 5 million to 15 million IU/m² and most preferably 7.5 million to 12.5 million IU/m².

The treatment may be continued until a clinical response is achieved or until intolerable side effects are encountered. The dosages of temozolomide and/or alpha interferon may be increased with each new treatment cycle, provided intolerable side effects are not encountered. The dosages may also be decreased, if intolerable side effects are encountered.

A common, but tolerable, side effect of temozolomide is nausea and vomiting. This can be alleviated by administering an anti-emetic in conjunction with the temozolomide. It is preferred that the anti-emetic Ondansetron be given p.o. in a dose of about 8 mg about 30 minutes before temozolomide administration. Of course other anti-emetics such as Haldol, Benadryl, and Ativan may also be used as needed.

A common, but usually tolerable, side effect of alpha interferon is flu-like symptoms. These can usually be alleviated with acetaminophen and other common aspirin-like medicines.

Of course, other forms of administration of both active ingredients, as they become available, are contemplated, such as by nasal spray, transdermally, by suppository, by sustained release dosage form, by IV injection, etc. Any form of administration will work so long as the proper dosages are delivered without destroying the active ingredient.

The effectiveness of treatment may be determined by controlled clinical trials. Patients having advanced cancer with measurable or evaluable tumors will be included in the study. A measurable tumor is one that can be measured in at least two dimensions such as a lung tumor surrounded by aerated lung, a skin nodule, or a superficial lymph node. An evaluable tumor in one that can be measured in one dimension such as a lung tumor not completely surrounded by aerated lung or a palpable abdominal or soft tissue mass that can be measured in one dimension. Tumor markers which have been shown to be highly correlated with extent of disease will also be considered to provide an evaluable disease, such as PSA for prostate cancer, CA-125 for ovarian cancer, CA-15-3 for breast cancer, etc.

The tumor will be measured or evaluated before and after treatment by whatever means provides the most accurate measurement, such as CT scan, MRI scan, Ultrasonography, etc. New tumors or the lack thereof in previously irradiated fields can also be used to assess the anti-tumor response. The criteria for evaluating response will be similar to that of the WHO Handbook of Reporting Results of Cancer Treatment, WHO Offset Publication 1979, 49-World Health Organization, Geneva. The following results are defined for uni- and bi-dimensionally measurable tumors.

Complete response: Complete disappearance of all clinically detectable malignant disease determined by two observations not less than four weeks apart.

Partial Response: (a) for bidimensionally measurable tumors, a decrease of at least 50% in the sum of the products of the largest perpendicular diameters of all measurable tumors as determined by two observations not less than four weeks apart. (b) for unidimensionally measurable tumors, a decrease by at least 50% in the sum of the largest diameters of all tumors as determined by two observations not less than four weeks apart. In cases where the patient has multiple tumors, It is not necessary for all tumors to have regressed to achieve a partial response as defined herein, but no tumor should have progressed and no new tumor should appear.

Stable disease: (a) for bidimensionally measurable tumors, less than a 50% decrease to less than a 25% increase in the sum of the products of the largest perpendicular diameters of all measurable tumors. (b) for unidimensionally measurable tumors, less than a 50% decrease to less than a 25 % increase in the sum of the diameters of all tumors. For (a) and (b) no new tumors should appear.

No clinical response, i.e. progressive disease in defined as an increase of more than 50% in the product of the largest perpendicular diameters for at least one bidimensionally measurable tumor, or an increase of more than 25 % in measurable dimension of at least one unidimensionally measurable tumor.

For patients having both uni- and bi-dimensionally measurable tumors, the overall response will be determined in accordance with the following table.

| Response in bidimensionally measurable disease | Response in unidimensionally measurable disease | Overall Response |
|---|---|---|
| PD | any | PD |
| Any | PD | PD |
| SD | SD or PR | SD |
| SD | CR | PR |
| PR | SD or PR or CR | PR |
| CR | SD or PR | PR |
| CR | CR | CR |
| Abbreviations: PD: Progressive Disease CR: Complete Response PR: Partial Response SD: Stable Disease | | |

Of course elimination or alleviation of other known signs or symptoms of advanced cancer, especially those listed previously can also be used to evaluate the effectiveness of this invention.

The advanced cancers should be evaluated, i.e. tumors measured, etc., no more than 14 days before the start of the treatment. These cancers should be reevaluated about 28 days after day 1 of administration of the first doses of temozolomide and alpha interferon. Twenty eight days after this initial administration another administration period may be performed, and evaluations performed 28 days after the start of this second cycle. The treatment cycles may be continued until a clinical response is achieved or unacceptable toxicity is encountered.

Another aspect of this invention is the treatment of advanced cancer with reduced side effects normally associated with temozolomide and alpha interferon. It is believed that this objective can be achieved by administration of lower doses of the two active ingredients or by shorter duration of dosing brought about by the synergistic effect of the combination.

The most serious side effect of temozolomide is hematologic toxicity. Dose limiting toxicity for temozolomide is defined herein as
CTC Grade 4 neutropenia (absolute neutrophil count, including bands, of less than 0.5 X 10³/ mm³) which is not resolved in five days or
CTC Grade 4 anemia (hemoglobin of less than 6.5 g/dl), or
CTC Grade 3 thrombocytopenia (platelet count of less than 50 X 10³/mm³) or
CTC Grade 4 thrombocytopenia(platelet count of less than 25 X 10³/mm³).

The most common side effects of alpha interferon are:
- flu-like syndrone
- Neurotoxicity, including neuropsychiatric, neurosensory, and neuromotor,
- Cardiopulmonary
- Gastrointestinal, including nausea, vomiting, and/or diarreha
- Hepatotoxicity, including elevations of bilirubin, transaminases, or alkaline phosphatase
- Nephrotoxicity.

## Claims

1. The use of temozolomide in the manufacture of a medicament for use in the treatment of cancer in a combination therapy in which alpha interferon is also to be administered.

2. The use of alpha interferon in the manufacture of a medicament for use in the treatment of cancer in a combination therapy in which temozolomide is also to be administered.

3. The use of Claim 1 or Claim 2 wherein the temozolomide is to be administered at a rate of from 50 to 400 mg per m² of the patient's body surface area per day for a period of from 2 to 10 days and the amount of alpha interferon to be administered is from 1 million to 25 million IU per m² of the patient's body surface area administered intravenously or subcutaneously 1 to 7 times per week.

4. The use of any preceding claim wherein the amount of temozolomide to be administered is from 75 to 300 mg per m² for a period of from 3 to 8 days and the amount of alpha interferon to be administered is from 5 million to 15 million IU per m² of the patient's body surface area administered intravenously or subcutaneously.

5. The use of any preceding claim wherein the amount of temozolomide to be administered is from 100 to 200 mg per m² of the patient's body surface area per day for a period of 5 days and the amount of alpha interferon to be administered is from 7.5 to 12.5 million IU per m² of the patient's body surface area administered intravenously or subcutaneously.

6. The use of any preceding claim wherein beginning 28 to 42 days after the first day of the temozolomide administration period, the temozolomide administrations are repeated.

7. The use of any preceding claim wherein beginning about 28 to 35 days after the first day of the temozolomide administration period, the temozolomide administrations are repeated.

8. The use of any preceding claim wherein the temozolomide is to be administered orally after the patient has fasted from food and liquids other than water for 4 hours before temozolomide administration and for 2 hours after temozolomide administration.

9. The use of any preceding claim wherein the temozolomide is to be administered orally for a period of from six days to six weeks.

10. The use of any preceding claim wherein the interferon is interferon 2b.

## Revendications

1. Utilisation d'un temozolomide dans la fabrication d'un médicament destiné à être utilisé dans le traitement du cancer, dans une thérapie de combinaison, dans laquelle on doit également administrer un interféron α.

2. Utilisation d'un interféron α dans la fabrication d'un médicament destiné à être utilisé dans le traitement du cancer, dans une thérapie de combinaison, dans laquelle on doit également administrer un temozolomide.

3. Utilisation selon la revendication 1 ou 2, dans laquelle on doit administrer le temozolomide en une proportion de 50 à 400 mg par m² de la surface corporelle du patient, par jour, sur une durée allant de 2 à 10 jours, et la quantité d'interféron α devant être administrée est comprise entre 1 million et 25 millions IU par m² de la surface corporelle du patient et est administrée par voie intraveineuse ou sous-cutanée 1 à 7 fois par semaine.

4. Utilisation selon l'une quelconque des revendications précédentes, dans laquelle la quantité de temozolomide devant être administrée est comprise entre 75 et 300 mg par m² sur une durée de 3 à 8 jours, et la quantité d'interféron α devant être administrée est comprise entre 5 millions et 15 millions IU par m² de la surface corporelle du patient et est administrée par voie intraveineuse ou sous-cutanée.

5. Utilisation selon l'une quelconque des revendications précédentes, dans laquelle la quantité de temozolomide devant être administrée est comprise entre 100 et 200 mg par m² de la surface corporelle du patient, par jour, sur une durée de 5 jours, et la quantité d'interféron α devant être administrée est comprise entre 7,5 millions et 12,5 millions IU par m² de la surface corporelle du patient et est administrée par voie intraveineuse ou sous-cutanée.

6. Utilisation selon l'une quelconque des revendications précédentes, dans laquelle en commençant 28 à 42 jours à compter du premier jour de la durée d'administration d'un temozolomide, on répète les administrations de temozolomide.

7. Utilisation selon l'une quelconque des revendications précédentes, dans laquelle en commençant environ 28 à 35 jours à compter du premier jour de la durée d'administration d'un temozolomide, on répète les administrations de temozolomide.

8. Utilisation selon l'une quelconque des revendications précédentes, dans laquelle on doit administrer un temozolomide par voie orale après que le patient n'a pas absorbé d'aliments et de liquides différents de l'eau 4 heures avant l'administration du temozolomide et 2 heures après l'administration du temozolomide.

9. Utilisation selon l'une quelconque des revendications précédentes, dans laquelle on doit administrer un temozolomide par voie orale sur une durée de 6 jours à 6 mois.

10. Utilisation selon l'une quelconque des revendications précédentes, dans laquelle l'interféron est un interféron 2b.

## Patentansprüche

1. Verwendung von Temozolomid zur Herstellung eines Medikaments zur Verwendung bei der Behandlung von Krebs in einer Kombinationstherapie, in der auch α-Interferon verabreicht wird.

2. Verwendung von α-Interferon zur Herstellung eines Medikaments zur Verwendung bei der Behandlung von Krebs in einer Kombinationstherapie, in der auch Temozolomid verabreicht wird.

3. Verwendung nach Anspruch 1 oder Anspruch 2, bei der das Temozolomid in einer Menge von 50 bis 400 mg pro m² der Körperoberfläche des Patienten pro Tag für einen Zeitraum von 2 bis 10 Tagen verabreicht wird, und die verabreichte Menge an α-Interferon 1 Million bis 25 Millionen IU pro m² der Körperoberfläche des Patienten beträgt und intravenös oder subkutan 1 bis 7 Mal pro Woche verabreicht wird.

4. Verwendung nach einem der vorhergehenden Ansprüche, bei der die verabreichte Menge an Temozolomid 75 bis 300 mg pro m² für einen Zeitraum von 3 bis 8 Tagen beträgt, und die verabreichte Menge an α-Interferon 5 Million bis 15 Millionen IU pro m² der Körperoberfläche des Patienten beträgt und intravenös oder subkutan verabreicht wird.

5. Verwendung nach einem der vorhergehenden Ansprüche, bei der die verabreichte Menge an Temozolomid 100 bis 200 mg pro m² Körperoberfläche des Patienten für einen Zeitraum von 5 Tagen beträgt, und die verabreichte Menge an α-Interferon 7,5 Million bis 12,5 Millionen IU pro m² der Körperoberfläche des Patienten ist und intravenös oder subkutan verabreicht wird.

6. Verwendung nach einem der vorhergehenden Ansprüche, bei der die Temozolomid-Verabreichungen beginnend 28 bis 42 Tage nach dem ersten Tag des Temozolomid-Verabreichungszeitraums wiederholt werden.

7. Verwendung nach einem der vorhergehenden Ansprüche, bei der die Temozolomid-Verabreichungen beginnend 28 bis 35 Tage nach dem ersten Tag des Temozolomid-Verabreichungszeitraums wiederholt werden.

8. Verwendung nach einem der vorhergehenden Ansprüche, bei der das Temozolomid oral verabreicht wird, nachdem der Patient sich Nahrungsmitteln und Flüssigkeiten außer Wasser 4 Stunden vor der Temozolomid-Verabreichung und 2 Stunden nach der Temozolomid-Verabreichung enthalten hat.

9. Verwendung nach einem der vorhergehenden Ansprüche, bei der das Temozolomid oral für einen Zeitraum von sechs Tagen bis sechs Wochen verabreicht wird.

10. Verwendung nach einem der vorhergehenden Ansprüche, bei der das Interferon Interferon 2b ist.
